# EUROPEAN PATENT APPLICATION

(11) **EP 4 813 418 A1**
(43) Date of publication of application: **30.09.2026**
(21) Application number: 24893216.2
(22) Date of filing: 04.11.2024
(51) Int. Cl.: A61M 5/178

(54) **LOADING AND UNLOADING METHOD FOR INJECTION NEEDLE ASSEMBLY, INJECTION PEN CAP, AND INJECTION PEN**

(30) Priority: 22.11.2023 CN 202311567580
(71) Applicant: Shanghai Innopac Medical Technology Co., Led., Shanghai 201605 (CN)
(72) Inventor: WU, Ding, Shanghai 201605 (CN); GUO, Rong, Shanghai 201605 (CN)
(74) Representative: karo IP
(86) International application number: PCT/CN2024/129580
(87) International publication number: WO 2025/108058

(57) **Abstract**

The present invention provides a method for mounting and dismounting an injection needle assembly, an injection pen cap, and an injection pen. The injection pen cap is detachably connected to an injection pen body and is capable of rotating relative to the injection pen body, and comprises a pen cap body. The pen cap body is provided, at a front end thereof, with a limiting cavity for mounting an injection needle assembly. When the injection needle assembly is mounted within the limiting cavity, the pen cap body is capable of rotating to drive a needle hub of the injection needle assembly to be connected to or disengaged from a front end of the injection pen body. When the needle hub is connected to the front end of the injection pen body, the pen cap body is capable, when being pulled off the injection pen body, of driving an outer needle cap of the injection needle assembly to disengage from the needle hub of the injection needle assembly; alternatively, the pen cap body is capable, when being engaged onto the injection pen body, of driving the outer needle cap of the injection needle assembly to be re-engaged with the needle hub of the injection needle assembly. The present invention is capable of preventing the user or handler from being pricked, and improves the safety performance during use of the injection needle assembly.

## Description

### Technical Field

The present invention belongs to the field of auto-injection technology, and specifically relates to a method for mounting and dismounting an injection needle assembly, an injection pen cap, and an injection pen.

### Background Art

An insulin pen is a common insulin injection device. For example, a front end of an insulin pen is provided with a needle, and the needle is provided with a cap covering it. At present, the vast majority of patients choose to use reusable insulin pens; reusable insulin pens can be used repeatedly; after the cartridge is used up, the cartridge can be replaced and used again; an insulin pen can be used for many years, even for a lifetime.

The current procedure for using an insulin pen is specifically as follows: 1, pulling off the pen cap; 2, unscrewing the cartridge holder; 3, retracting the piston rod; 4, installing the cartridge (loading into the cartridge holder); 5, mounting the cartridge holder onto the injection pen body; 6, mounting the needle; 7, removing the outer needle cap and the inner needle cap from the needle. Among the above steps, the step of pulling off the inner needle cap is the most dangerous; the inner needle cap is immediately adjacent to the needle, and the volume of the inner needle cap is very small, such that the user can easily be pricked. Furthermore, during mounting of the injection needle assembly, the needle cap may easily be knocked off, which can easily prick the user. Similarly, when use is completed and the needle needs to be replaced, accidents of pricking the user's hand can easily occur during the process of removing the needle.

### Summary of the Invention

Aimed at the problems existing in the above background art, an object of the present invention is to provide a method for mounting and dismounting an injection needle assembly, an injection pen cap, and an injection pen which have good safety performance and are convenient to use.

The technical solution adopted by the present invention is as follows:
An injection pen cap, which is detachably connected to an injection pen body and is capable of rotating relative to the injection pen body, comprising a pen cap body, wherein the pen cap body is provided, at a front end thereof, with a limiting cavity for mounting an injection needle assembly; when the injection needle assembly is mounted within the limiting cavity, the pen cap body is capable of rotating to drive a needle hub of the injection needle assembly to be connected to or disengaged from a front end of the injection pen body; when the needle hub is connected to the front end of the injection pen body, the pen cap body is capable, when being pulled off the injection pen body, of driving an outer needle cap of the injection needle assembly to disengage from the needle hub of the injection needle assembly, or the pen cap body is capable, when being engaged onto the injection pen body, of driving the outer needle cap of the injection needle assembly to be re-engaged with the needle hub of the injection needle assembly.

Further, the pen cap body is provided, at a position of the limiting cavity, with a pen cap lid for opening or closing the limiting cavity.

Further, one side of the pen cap lid is hingedly connected to the pen cap body, and the other side of the pen cap lid is engagedly connected to the pen cap body.

Further, the pen cap body is provided, on an engagement side thereof, with a recess for manually opening the pen cap lid.

Further, the pen cap lid is provided with a marking indicating a rotational connection direction.

Further, a structural shape of the limiting cavity is consistent with a structural shape of an outer surface of the outer needle cap of the injection needle assembly and is capable of cooperating with the outer needle cap to restrict rotation of the outer needle cap.

Further, a rear end surface of the limiting cavity cooperates with an end surface of the outer needle cap to axially limit the outer needle cap.

An injection pen, comprising an injection pen body, wherein the above-described injection pen cap is detachably mounted on the injection pen body, and the injection pen cap is capable of detachably connecting the injection needle assembly to the injection pen body.

A method for mounting and dismounting an injection needle assembly, using the above-described injection pen, the method comprising the following specific steps:
S1, pulling the injection pen cap off the injection pen body, opening the pen cap lid, placing the injection needle assembly into the limiting cavity, and closing the pen cap lid;
S2, engaging the injection pen cap having the injection needle assembly mounted therein onto the injection pen body, and rotating the injection pen cap to tighten it according to the marking, at which time the needle hub of the injection needle assembly is connected to the front end of the injection pen body;
S3, pulling the injection pen cap off the injection pen body, at which time the injection pen cap drives the outer needle cap of the injection needle assembly to disengage from the needle hub;
S4, removing the inner needle cap from the needle hub so as to perform medication administration with the injection pen body, and after completion of medication administration, re-engaging the injection pen cap carrying the outer needle cap onto the injection pen body, and rotating the injection pen cap to loosen it according to the marking, at which time the needle hub carrying the injection needle is disengaged from the front end of the injection pen body and is re-engaged by the outer needle cap;
S5, pulling the injection pen cap off the injection pen body, at which time the injection pen cap drives the injection needle assembly not containing the inner needle cap to leave the injection pen body;
S6, opening the pen cap lid, removing the injection needle assembly not containing the inner needle cap, then closing the pen cap lid, and engaging the injection pen cap onto the injection pen body to await next use.

Further, the needle hub of the injection needle assembly is threadedly connected to the front end of the injection pen body.

Compared with the prior art, the significant advantages of the present invention include: the injection pen cap not only serves a dust-proof function but also serves the function of mounting and dismounting the injection needle assembly; the injection needle is not contacted throughout the mounting and dismounting of the injection needle assembly, thereby preventing the user or handler from being pricked and improving the safety performance during use of the injection needle assembly.

### Brief Description of the Drawings

FIG. 1 is a schematic structural view of the injection pen cap of the present invention;
FIG. 2 is a schematic structural view of the present invention after the pen cap lid is opened;
FIG. 3 is a schematic cross-sectional structural view of FIG. 2;
FIG. 4 is a schematic structural view of the injection pen cap of the present invention with the injection needle assembly mounted therein;
FIG. 5 is a schematic structural view of the injection pen of the present invention;
FIG. 6 is a schematic structural view of the injection pen of the present invention with the pen cap removed;
FIG. 7 is a schematic flow view of the mounting of the injection needle assembly of the present invention;
FIG. 8 is a schematic flow view of the removal of the injection needle assembly of the present invention.

### Reference signs:

1 - injection pen cap; 11 - pen cap body; 12 - limiting cavity; 121 - recessed strip; 122 - rear end surface; 13 - pen cap lid; 14 - recess; 15 - marking; 16 - latch; 17 - latch opening; 2 - injection needle assembly; 21 - needle hub; 22 - outer needle cap; 3 - injection pen body.

### Detailed Description of Embodiments

The present invention will be further described below in conjunction with specific embodiments, but the present invention is not limited to these specific embodiments. Those skilled in the art should recognize that the present invention encompasses all alternative solutions, modifications, and equivalents that may be included within the scope of the claims.

In the description of the present invention, it should be understood that the terms "center", "longitudinal", "transverse", "length", "width", "thickness", "upper", "lower", "front", "rear", "left", "right", "vertical", "horizontal", "top", "bottom", "inner", "clockwise", "counterclockwise" and the like indicate orientations or positional relationships based on the orientations or positional relationships shown in the drawings, and are merely for facilitating description of the present invention and simplifying description, rather than indicating or implying that the referred device or element must have a specific orientation or be constructed and operated in a specific orientation, and therefore cannot be construed as limiting the present invention. Furthermore, the terms "first" and "second" are used for descriptive purposes only and cannot be construed as indicating or implying relative importance or implicitly indicating the number of the technical features indicated. Thus, features defined with "first" or "second" may explicitly or implicitly include one or more such features. In the description of the present invention, unless otherwise specified, "a plurality of" means two or more, unless otherwise expressly defined.

In the present invention, unless otherwise expressly stipulated and defined, the terms "mount", "couple", "connect", "fix" and the like should be understood in a broad sense. For example, they may refer to a fixed connection, a detachable connection, or an integral connection; may be a mechanical connection or an electrical connection; may be a direct connection, or may be an indirect connection through an intermediate medium, or may be a communication between interiors of two elements. For those of ordinary skill in the art, the specific meanings of the above terms in the present invention can be understood according to specific circumstances.

In the present invention, unless otherwise expressly stipulated and defined, a first feature being "above" or "below" a second feature may include the first and second features being in direct contact, or may include the first and second features being not in direct contact but in contact through another feature therebetween. Moreover, the first feature being "above", "over" and "on top of" the second feature includes the first feature being directly above and obliquely above the second feature, or merely indicates that the horizontal height of the first feature is higher than that of the second feature. The first feature being "below", "under" and "beneath" the second feature includes the first feature being directly below and obliquely below the second feature, or merely indicates that the horizontal height of the first feature is less than that of the second feature.

### Embodiment 1

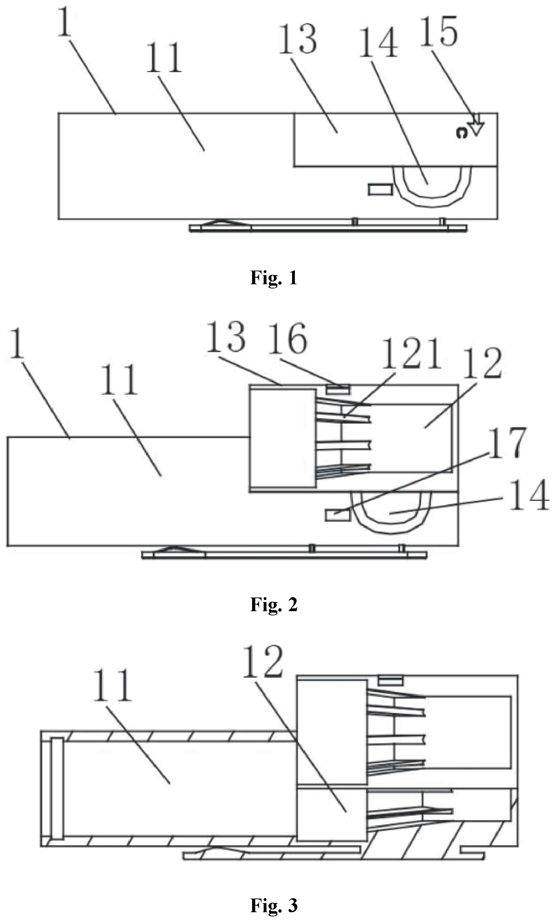

Referring to FIGS. 1-4, the present embodiment provides an injection pen cap 1, which is detachably connected to an injection pen body and is capable of rotating relative to the injection pen body, the injection pen cap 1 comprising a pen cap body 11; the pen cap body 11 is provided, at a front end thereof, with a limiting cavity 12 for mounting an injection needle assembly 2; when the injection needle assembly 2 is mounted within the limiting cavity 12, the pen cap body 11 is capable of rotating to drive a needle hub of the injection needle assembly 2 to be connected to or disengaged from a front end of the injection pen body; when the needle hub is connected to the front end of the injection pen body, the pen cap body 11 is capable, when being pulled off the injection pen body, of driving an outer needle cap of the injection needle assembly 2 to disengage from the needle hub of the injection needle assembly 2, or the pen cap body 11 is capable, when being engaged onto the injection pen body, of driving the outer needle cap of the injection needle assembly 2 to be re-engaged with the needle hub of the injection needle assembly 2.

In the present embodiment, the pen cap body 11 is provided, at a position of the limiting cavity 12, with a pen cap lid 13 for opening or closing the limiting cavity 12. One side of the pen cap lid 13 is hingedly connected to the pen cap body 11, and the other side of the pen cap lid 13 is engagedly connected to the pen cap body 11; specifically, a latch 16 is provided on the pen cap lid 13, the pen cap body 11 is provided, at a position corresponding to the latch 16, with a latch opening 17, and the latch 16 and the latch opening 17 cooperate for engagement. The pen cap body 11 is provided, on the engagement side, with a recess 14 for manually opening the pen cap lid 13; specifically, a shape of the recess 14 is similar to a shape of a front end of a thumb, so as to facilitate flicking the pen cap lid 13 to release the latch 16 from the latch opening 17, thereby opening the pen cap lid 13. The pen cap lid 13 is provided with a marking 15 indicating a rotational connection direction; specifically, the marking 15 may be affixed onto a surface of the pen cap lid 13, or may be engraved on the pen cap lid 13.

In the present embodiment, a structural shape of the limiting cavity 12 is consistent with a structural shape of an outer surface of the outer needle cap of the injection needle assembly 2 and is capable of cooperating with the outer needle cap to restrict rotation of the outer needle cap. Specifically, a surface of the outer needle cap is provided with protruding ridges, and an inner wall surface of the limiting cavity 12 is provided with recessed strips 121 cooperating with the ridges to restrict rotation; shapes of the ridges and the recessed strips 121 are consistent, and the recessed strips are capable of wrapping around the ridges, such that the limiting cavity 12 embraces the outer needle cap, thereby restricting rotation of the outer needle cap. A rear end surface 122 of the limiting cavity 12 cooperates with an end surface of the outer needle cap to axially limit the outer needle cap; through end-surface cooperation, the axial movement of the outer needle cap is restricted, such that the pen cap body 11 is capable, when being pulled off, of removing the outer needle cap from the needle hub.

The injection pen cap of the present invention serves not only a dust-proof function but also the function of mounting and dismounting the injection needle assembly 2; the injection needle is not contacted throughout the mounting and dismounting of the injection needle assembly 2, thereby preventing the user or handler from being pricked and improving the safety performance during use of the injection needle assembly 2.

### Embodiment 2

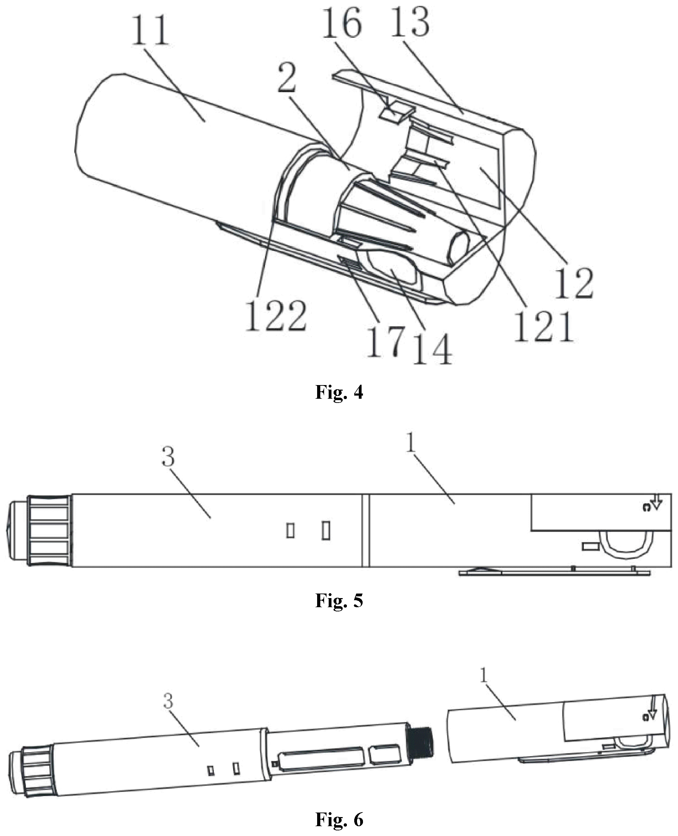

Referring to FIGS. 5 and 6, the present embodiment provides an injection pen, comprising an injection pen body 3, the injection pen cap 1 according to Embodiment 1 being detachably mounted on the injection pen body 3, and the injection pen cap 1 being capable of detachably connecting the injection needle assembly 2 to the injection pen body 3.

In an initial state, the injection needle assembly 2 is not mounted on the injection pen body 3; when use is required, the injection needle assembly 2 is connected to the injection pen body 3 via the injection pen cap 1; and after completion of use, the injection needle assembly 2 not containing the inner needle cap is removed via the injection pen cap 1.

The structure and function of the injection pen cap 1 can be referred to as described in Embodiment 1.

### Embodiment 3

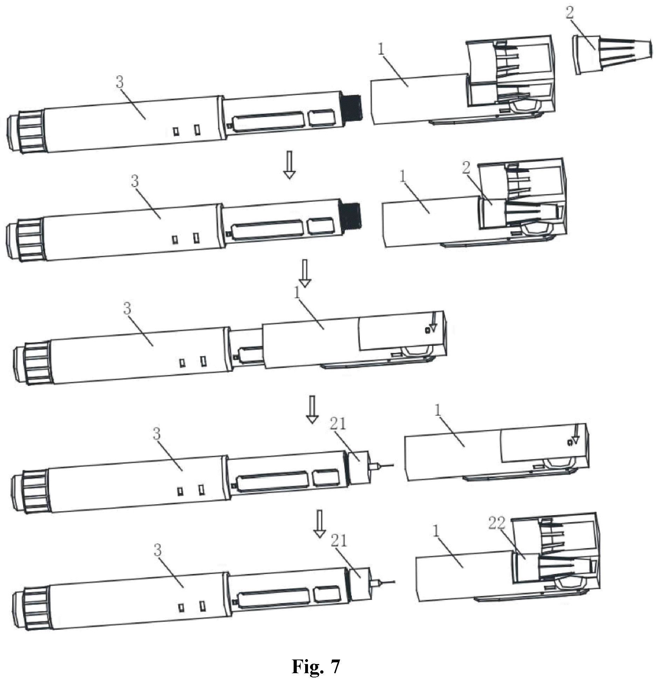

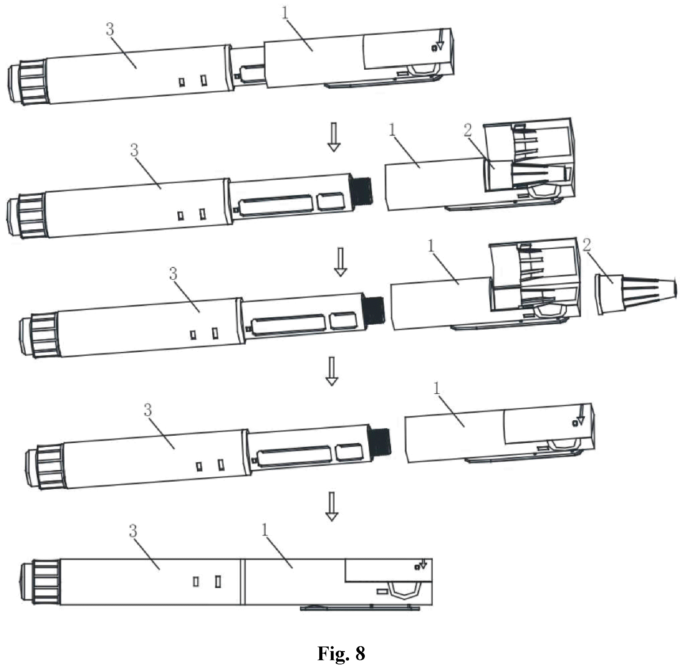

Referring to FIGS. 7 and 8, the present embodiment provides a method for mounting and dismounting an injection needle assembly, using the injection pen according to Embodiment 2, the method comprising the following specific steps:
S1, pulling the injection pen cap 1 off the injection pen body 3, opening the pen cap lid 13, placing the injection needle assembly 2 into the limiting cavity 12, and closing the pen cap lid 13;
S2, engaging the injection pen cap 1 having the injection needle assembly 2 mounted therein onto the injection pen body 3, and rotating the injection pen cap 1 to tighten it according to the marking 15, at which time the needle hub 21 of the injection needle assembly 2 is connected to the front end of the injection pen body 3;
S3, pulling the injection pen cap 1 off the injection pen body 3, at which time the injection pen cap 1 drives the outer needle cap 22 of the injection needle assembly 2 to disengage from the needle hub 21; that is, the outer needle cap 22 remains within the limiting cavity 12 of the injection pen cap 1;
S4, removing the inner needle cap (not shown in the figures) from the needle hub 21 so as to perform medication administration with the injection pen body 3, and after completion of medication administration, re-engaging the injection pen cap 1 carrying the outer needle cap 22 onto the injection pen body 3, and rotating the injection pen cap 1 to loosen it according to the marking 15, at which time the needle hub 21 carrying the injection needle is disengaged from the front end of the injection pen body 3 and is re-engaged by the outer needle cap 22;
S5, pulling the injection pen cap 1 off the injection pen body 3, at which time the injection pen cap 1 drives the injection needle assembly 2 not containing the inner needle cap to leave the injection pen body 3;
S6, opening the pen cap lid 13, removing the injection needle assembly 2 not containing the inner needle cap, then closing the pen cap lid 13, and engaging the injection pen cap 1 onto the injection pen body 3 to await next use; the inner needle cap and the used injection needle assembly 2 not containing the inner needle cap can be discarded into a designated waste bin.

In the present embodiment, the needle hub 21 of the injection needle assembly 2 is threadedly connected to the front end of the injection pen body 3.

A cartridge may be mounted within the injection pen body 3, in which case the needle hub 21 is connected to the front end of the injection pen body 3. Alternatively, the cartridge may also be directly connected to the front end of the injection pen body 3, in which case the needle hub 21 is connected to a front end of the cartridge.

According to the present invention, the injection pen cap serves the function of mounting and dismounting the injection needle assembly 2; the injection needle is not contacted throughout the mounting and dismounting of the injection needle assembly 2, thereby preventing the user or handler from being pricked and improving the safety performance during use of the injection needle assembly 2.

## Claims

1. An injection pen cap, which is detachably connected to an injection pen body (3) and is capable of rotating relative to the injection pen body (3), the injection pen cap comprising a pen cap body (11), wherein the pen cap body (11) is provided, at a front end thereof, with a limiting cavity (12) for mounting an injection needle assembly (2); when the injection needle assembly (2) is mounted within the limiting cavity (12), the pen cap body (11) is capable of rotating to drive a needle hub (21) of the injection needle assembly (2) to be connected to or disengaged from a front end of the injection pen body (3); when the needle hub (21) is connected to the front end of the injection pen body (3), the pen cap body (11) is capable, when being pulled off the injection pen body (3), of driving an outer needle cap (22) of the injection needle assembly (2) to disengage from the needle hub (21) of the injection needle assembly (2), or the pen cap body (11) is capable, when being engaged onto the injection pen body (3), of driving the outer needle cap (22) of the injection needle assembly (2) to be re-engaged with the needle hub (21) of the injection needle assembly (2).

2. The injection pen cap according to claim 1, wherein the pen cap body (11) is provided, at a position of the limiting cavity (12), with a pen cap lid (13) for opening or closing the limiting cavity (12).

3. The injection pen cap according to claim 2, wherein one side of the pen cap lid (13) is hingedly connected to the pen cap body (11), and the other side of the pen cap lid (13) is engagedly connected to the pen cap body (11).

4. The injection pen cap according to claim 3, wherein the pen cap body (11) is provided, on an engagement side thereof, with a recess (14) for manually opening the pen cap lid (13).

5. The injection pen cap according to claim 2, wherein the pen cap lid (13) is provided with a marking (15) indicating a rotational connection direction.

6. The injection pen cap according to claim 1, wherein a structural shape of the limiting cavity (12) is consistent with a structural shape of an outer surface of the outer needle cap (22) of the injection needle assembly (2) and is capable of cooperating with the outer needle cap (22) to restrict rotation of the outer needle cap (22).

7. The injection pen cap according to claim 6, wherein a rear end surface (122) of the limiting cavity (12) cooperates with an end surface of the outer needle cap (22) to axially limit the outer needle cap (22).

8. An injection pen, comprising an injection pen body (3), wherein the injection pen cap (1) according to any one of claims 1 to 7 is detachably mounted on the injection pen body (3), and the injection pen cap (1) is capable of detachably connecting the injection needle assembly (2) to the injection pen body (3).

9. A method for mounting and dismounting an injection needle assembly, using the injection pen according to claim 8, the method comprising the following steps:
S1, pulling the injection pen cap (1) off the injection pen body (3), opening the pen cap lid (13), placing the injection needle assembly (2) into the limiting cavity (12), and closing the pen cap lid (13);
S2, engaging the injection pen cap (1) having the injection needle assembly (2) mounted therein onto the injection pen body (3), and rotating the injection pen cap (1) to tighten it according to the marking (15), at which time the needle hub (21) of the injection needle assembly (2) is connected to the front end of the injection pen body (3);
S3, pulling the injection pen cap (1) off the injection pen body (3), at which time the injection pen cap (1) drives the outer needle cap (22) of the injection needle assembly (2) to disengage from the needle hub (21);
S4, removing an inner needle cap from the needle hub (21) so as to perform medication administration with the injection pen body (3), and after completion of medication administration, re-engaging the injection pen cap (1) carrying the outer needle cap (22) onto the injection pen body (3), and rotating the injection pen cap (1) to loosen it according to the marking (15), at which time the needle hub (21) carrying the injection needle is disengaged from the front end of the injection pen body (3) and is re-engaged by the outer needle cap (22);
S5, pulling the injection pen cap (1) off the injection pen body (3), at which time the injection pen cap (1) drives the injection needle assembly (2) not containing the inner needle cap to leave the injection pen body (3);
S6, opening the pen cap lid (13), removing the injection needle assembly (2) not containing the inner needle cap, then closing the pen cap lid (13), and engaging the injection pen cap (1) onto the injection pen body (3) to await next use.

10. The method for mounting and dismounting an injection needle assembly according to claim 9, wherein the needle hub (21) of the injection needle assembly (2) is threadedly connected to the front end of the injection pen body (3).
